# EUROPEAN PATENT SPECIFICATION

(11) **EP 0 671 174 B1**
(45) Date of publication and mention of the grant of the patent: **02.02.2005**
(21) Application number: 95301467.7
(22) Date of filing: 07.03.1995
(51) Int. Cl.: A61K 33/04

(54) **Formulation for increasing the weight of livestock, comprising an ionophore and selenium**
Zusammensetzung zur Gewichtserhöhung von Vieh, die ein Ionophore und Selenium enthält
Composition pour augmenter le poids du bétail, contenant un ionophore et du sélénium

(30) Priority: 08.03.1994 AU PM430894
(43) Date of publication of application: 13.09.1995
(73) Proprietor: ELI LILLY AND COMPANY, Indianapolis, Indiana 46285 (US)
(72) Inventor: Lowe, Lionel Barry, Dural, New South Wales 2158 (AU)
(74) Representative: Burnside, Ivan John

(56) References cited:
- L.S. JENSEN: "Effect of monensin on response of chicks to deficient and toxic levels of dietary selenium" POULTRY SCIENCE, vol. 65, no. 6, 1986, pages 1163-1166, XP002084626
- A. RAMISZ: "The influence of monensin and selenium on weight gain and coccidiosis in lambs" COLLOQ. INRA, vol. 49, no. COCCIDIA INTEST. COCCIDIOMORPHS, 1989, pages 431-434, XP002084627
- N.D. COSTA: "Effects of monensin controlled release capsules and selenium pellets on liveweight gain and tissue selenium" JOURNAL OF DAIRY SCIENCE, vol. 77, no. suppl. 1, 1994, page 134 XP002084628
- J.W. SPEARS: "IONOPHORES AND NUTRIENT DIGESTION AND ABSORPTION IN RUMINANTS" J NUTR 120 (6). 1990., pages 632-638, XP002084629
- J.H. WANG: "Effects of monensin on the bioavailability and elimination of selenium from blood in lambs" JOURNAL OF VETERINARY PHARMACOLOGY AND THERAPEUTICS, vol. 120, no. 6, 1994, pages 378-385, XP002084630
- N.D. COSTA: "Monensin and narasin increase selenium and zinc absorption in steers" TRACE ELEM. MAN ANIM. -- TEMA 5, PROC. INT. SYMP., 5TH (1985), MEETING DATE 1984, 472-4. EDITOR(S): MILLS, C. F.; BREMNER, I.; CHESTERS, J. K. PUBLISHER: CAB, FARNHAM ROYAL, SLOUGH, UK., XP002084631
- P.H. ANDERSON: "Effect of monensin on the selenium status of sheep" VETERINARY RECORD, vol. 113, no. 21, 1983, page 498 XP002084632

## Description

### Technical Field

This invention relates to a formulation for increasing the weight of livestock, method for increasing the liveweight gain in livestock and a method of increasing selenium absorption in livestock.

### Background Art

Mineral nutrients are required for most of the fundamental processes of life. Mineral nutrients are divided into two groups, major elements and trace elements. Greater quantities of the major elements are required compared to the trace elements.

Selenium is a trace element and has been thought to be widely distributed throughout the body tissues. Selenium is a necessary component of glutathione peroxidase, the enzyme responsible for reducing hydrogen peroxide and organic peroxides to alcohol and water. Selenium is also thought to play a role in the function of leukocyte acid phosphatase, glucuronidase, electron transfer and nonheme iron proteins.

The main site of absorption of selenium is the duodenum. The amount of selenium in the diet is critical. An excess of selenium leads to alkali disease and blind staggers of grazing livestock. A deficiency of selenium may cause heart disease and increase cancer. A classical sign of selenium deficiency is nutritional muscular dystrophy.

Selenium deficiency has also been shown to effect the incidence of mastitis in dairy cattle.

Ionophores such as monensin improve the efficiency of production in growing ruminants. Part of this production effect can be attributed to alteration of rumen function leading to increased molar proportions of propionate relative to acetate, and to an increase in the apparent whole-body retention of selenium. Anderson, P.H., Berrett, S., Catchpole, J., Gregory, M.W. and Brown, D.C. (1983) Veterinary Record, 113, 498 showed that ewes given sodium monensin as a coccidiostat had significantly higher glutathione peroxidase activity in their red blood cells than control ewes fed a basal diet of low selenium concentration.

### Objects of the Invention

It is an object of this invention to provide a formulation for increasing the weight of livestock and a method of increasing liveweight gain in livestock.

It is a further object of this invention to provide a method of increasing selenium absorption in livestock.

### Disclosure of the Invention

The present inventor has found that a formulation comprising an ionophore and selenium has a synergistic effect in increasing the liveweight gain in livestock.

The present invention provides a formulation for increasing weight of livestock comprising an ionophore, selenium and a veterinary acceptable carrier, diluent, excipient and/or adjuvant.

The present invention also provides a method of increasing liveweight gain in livestock comprising, administering to said livestock an effective amount of a formulation of the invention.

The present invention further provides a method of increasing selenium absorption in livestock comprising, administering to said livestock an effective amount of an ionophore.

The ionophore used in the formulation is generally a carboxylic ionophore or polyether antibiotic. The polyether antibiotics which can be employed include those produced by the Streptomyces genus or microorganisms. They are characterised by comprising a multiplicity of cyclic ethers in their structures. The class is reviewed in *Kirk-Othmer: Encyclopedia of Chemical Technology,* Vol. 3, Third Edition (John Wiley & Sons, 1978), Page 47 *et seq*.; in *Kirk-Othmer: Encyclopedia of Chemical Technology*, Vol. 3, Fourth Edition (John Wiley & Sons, 1992), Page 306 *et seq*.; in *Annual Reports in Medical Chemistry* Volume 10 (Academic Press, N.Y. 1878), page 246 *et seq.*; and in *J. Chrom. Lib*., Volume 15 (Elsevier Scientific Publishing Co., N.Y. 1978), page 488 *et seq*.

Representative of the polyether antibiotics to be employed in the formulation of this invention include ruminal propionate enhancers such as monensin (including the various factors A, B, and C, and the alkali metal salts, for instance monensin sodium, and the various esters thereof), narasin, lasalocid and salinomycin.

An especially preferred polyether to be utilised accordmg to this invention is monensin, a compound widely used in the improvement of feed utilisation in ruminants (see U.S. Patent No. 3,839,557). As used herein, "monensin" includes the various active factors, the salts such as monensin sodium, and the monensin esters such as carbamate esters and the like.

Generally the amount of ionophore used in the formulation ranges from 0.5 to 60wt%, preferably 0.5 to 40wt% based on the total amount of formulation. Generally 75 to 250mg of monensin is used, preferably 125 to 300mg or 125 to 200mg is used per head per day for cattle.

Selenium, selenium compounds including selenium salts may be used in the formulation for example selenium dioxide, selenium oxyhalide, selenium bromide, selenium sulfide, selenides, selenates for example, barium selenate, or selenites. Elemental selenium and/or barium selenate is preferably used in the formulation.

Generally the amount of selenium used in the formulation ranges from 0.01 to 2wt% based on the total amount of formulation. Usually 1 to 10mg of selenium, preferably 1 to 10mg of selenium is used per day, more preferably 0.02 to 0.026ppm is used.

The veterinary acceptable carrier or excipient is for example sodium citrate; dicalcium phosphate; binders, for example, alginate, gelatin, carboxymethylcellulose or polyvinylpyrrolidone; humectants, for example, glycerol; fillers and extenders, for example, sucrose, lactose, starch, glucose, mannitol or silicic acid; solution retarders, for example, paraffin; disintegrating agents, for example, calcium carbonate, agar-agar, algininic acid, potato starch, tapioca starch, sodium carbonate; absorption accelerators, for example, quaternary ammonium compounds; wetting agents, for example, cetyl alcohol, glycerol monostearate; absorbents, for example, kaolin, bentonite; lubricants, for example, magnesium stearate, solid polyethylene glycol, sodium lauryl sulphate, talc, or calcium stearate.

Examples of a veterinary acceptable adjuvant are preserving, wetting, emulsifying or dispensing agents. Some examples of these agents are lecithin, hexaglycerol distearate (HGDS), sucrose esters, polyoxyethylene stearate, heptadecaethyleneoxycetanol, polyoxyethylene sorbitol monooleate, polyoxyethylene sorbitan monooleate, ethyl or n-propyl p-hydroxybenzoate.

Examples of a veterinary acceptable diluent are cottonseed oil, groundnut oil, castor oil, olive oil, sesame oil, corn germ oil, glycerol, glycerolformal, tetrahydrofurfuryl alcohol, polyethylene glycol, fatty acid esters of sorbitan, benzyl alcohol, propylene glycol, ethyl alcohol, isopropyl alcohol, ethyl carbonate, ethyl acetate, benzyl benzoate, 1,3-butylene glycol, corn meal, rice hulls or soy meal.

Generally the amount of veterinary carrier, diluent, excipient and/or adjuvant is 40 to 99wt%, preferably 60 to 99wt% based on the total amount of formulation. Usually 95 to 99wt% of veterinary carrier, diluent, excipient and/or adjuvant is used.

The formulation may be administered directly into feed or by way of a controlled release capsule (CRC) which is inserted into the rumen of the livestock. Examples of suitable capsules may be found in Australian Patent No. 650 113, (filed on 1 April 1992 and assigned to Eli Lilly and Company), Australian Patent Application No. 72867/94 (divisional application based on AU 650 113 with an effective filing date of 1 April 1992 and assigned to Eli Lilly and Company), U.S. Patent No. 5,277,912 (filed on 6 April 1992 and assigned to Eli Lilly and Company) and United States Serial No. 08/163,842 (divisional application based on US 5,277,912 and filed on 7 December 1993 and assigned to Eli Lilly and Company).

Generally the amount of formulation administered to a livestock varies on the liveweight of the livestock. Typically 0.5 to 10wt% of formulation is administered to the livestock, preferably 0.75 to 2.5wt% is administered, more preferably 1.25 to 2wt% is administered. The wt% is based on the total liveweight of the livestock. The formulation may be administered daily, weekly, monthly or yearly, preferably the formulation is administered daily.

The formulation may be administered to any livestock, for example cattle, sheep or any other animal. If the formulation is administered as a capsule, the size of the capsule may be adapted to suit any sized animal or ruminant species.

The formulation may comprise further additives such as a glycopeptide antibiotic, an anthelmintic, an ectoparasiticide, a non-ionic surfactant or a silicone antifoam agent.

Examples of a glycopeptide antibiotic are actaplanin, avoparcin, A35512, A477, ristocetin, vancomycin, and related glycopeptides.

Examples of an anthelmintic are antimony, avermectins, potassium tartrate, bephenium hydroxy naphthoate, bithionol, chloroquine, dichlorophen, diethylcarbamazine citrate, hexylresorcinol, hycanthone mesylate, lucanthone hydrochloride, mebendazole, niclosamide, niridazole, piperazine citrate, pyrantel pamoate, pyrvinium pamoate, quinacrine hydrochloride, stibocaptate, stibophen, tetrachloroethylene, fenbendazole, thiabendazole, phenothiazine, hexachloroethane, carbon disulphide or benzimidazole. An especially preferred anthelmintic is benzimidazole, fenbendazole or ivermectin. Examples of suitable a benzimidazole include thiabendazole, albendazole, cambendazole, fenbendazole, mebendazole, oxfendazole, or oxibendazole.

Examples of an ectoparasiticide are organophosphates and carbamates, avermectins, levamisole or sodium thiacetarsamide.

Examples of a non ionic surfactant are alcohol ethoxylates. Generally the ethoxylates are of octyl-, nonyl- and dodecyl phenol, natural and synthetic alcohols, saturated and unsaturated fatty acids and both block and random copolymers. Alcohol ethoxylates such as those of the Teric® series of the Pluronic® PE series of mixtures thereof are preferred. An especially preferred non ionic surfactant is Teric® 12A23, which is lauryl (dodecanol) condensed with 23 moles of ethylene oxide.

Examples of a silicone anti foam agent are aqueous or anhydrous, preferably anhydrous. The silicone anti foam agents may be a mixture of dimethyl silicones or silicone glycol, such as those of Gensil® series or the Rhodorsil® series. An especially preferred silicone anti foam agent is Gensil® 800 or Silbione® 70 451 or BC 403 (or similar, Basilidon).

### Best Mode For Carrying Out The Invention

A formulation comprising 0.5 to 60wt% ionophore, 0.01 to 2wt% selenium and 93 to 99.49wt% veterinary acceptable carrier, diluent, excipient and/or adjuvant has been found to increase the liveweight gain of livestock. A preferred formulation comprises 0.5 to 5wt% ionophore, 0.01 to 2wt% selenium and 93 to 99.49wt% veterinary acceptable carrier, diluent, excipient and/or adjuvant. The formulation usually comprises 75 to 250mg monensin or narasin, 0.01 to 0.03ppm selenium and 95 to 99wt% veterinary acceptable carrier, diluent, excipient and/or adjuvant. A preferred formulation comprises 125 to 350mg monensin or narasin, 0.02 to 0.026ppm selenium and 95 to 99wt% veterinary acceptable carrier, diluent, excipient and/or adjuvant. Alternatively another preferred formulation comprises 125 to 200mg monensin or narasin, 0.02 to 0.026ppm selenium and 95 to 99wt% veterinary acceptable carrier, diluent, excipient and/or adjuvant.

When 0.5 to 10wt% of any of the above formulations is administered daily, the liveweight gain in livestock is increased. Preferably 0.75 to 2.5wt%, more preferably 1.25 to 2wt% of the above formulation increases the liveweight gain in livestock. The wt% is based on the total liveweight of the livestock.

Also 0.5 to 10wt% of any of the above formulations may also increase the amount of selenium absorbed from the diet. Generally the amount of selenium absorbed is doubled using the formulation. Preferably 0.75 to 2.5wt%, more preferably 1.25 to 2wt% of the above formulation increases the amount of selenium absorbed from the diet. The wt% is based on the total liveweight of the livestock.

A preferred amount of ionophore used to increase selenium absorption in livestock comprises 75 to 250mg monensin or narasin, more preferably 125 to 200mg monensin or narasin administered daily. The ionophore may be administered directly into feed or by way of a controlled release capsule (CRC) which is inserted into the rumen of the livestock.

The following examples illustrate the typical formulations which lead to an increase in the liveweight gain in livestock and an increase in the amount of selenium absorbed from the diet.

### Example 1

Forty eight yearling steers, of mean liveweight 285.2 ± 3.7 kg, were purchased at market, and allocated evenly to one of four treatment groups: control (blank CRC), monensin (monensin CRC), selenium (blank CRC and Se pellets), or monensin, plus selenium (monensin CRC and Se pellets), according to ranked liveweight. All of the steers were fed hay (60%) and lupins (40%) at a rate of 2.8% of mean liveweight for the group per day for 70 days and then 3% for a further 30 days, giving a total of 100 days on feedlot. This ration contained at least 80 MJ ME, 14% protein, and less than 0.02 ppm Se. All animals were weighed every ten days. All of the steers were slaughtered at a commercial abattoir, CRCs were recovered and measured and samples of liver and diaphragm muscle obtained from each steer. The CRCs released monensin at a rate of 199.4 ± 4.6 mg per day over the 100 days of feeding. Liveweight (LW) gain (in kg) over the 100 days and tissue Se concentrations (ppb wet weight of tissue) are presented in the table. All values are means ± SEMs for 12 animals per treatment. Values with different superscripts are significantly different at P < 0.01 and at P < 0.05 by ANOVA.

| Treatment | LW Gain | Liver Se | Muscle Se |
|---|---|---|---|
| Control | 72.9 ± 10.1 | 222.5 ± 15.7^{a} | 70.8 ± 6.5^{a} |
| Monensin | 77.1 ± 7.7 | 253.8 ± 12.0^{a} | 75.7 ± 6.5^{a} |
| Selenium | 80.0 ± 9.0 | 314.9 ± 16.1^{a,b} | 95.2 ± 4.7^{a} |
| Monensin+Se | 94.7 ± 9.2 | 330.6 ± 13.2^{a,b} | 103.2 ± 7.9^{a,b} |

Steers from the monensin + Se group showed a 30% increase in liveweight gain relative to the control group, and a significant increase in muscle Se compared to all other treatments. Monensin and Se were more effective in combination than singly at increasing liveweight gain and tissue Se concentrations in steers fed on low Se rations.

### Example 2

One Guernsey and three Jersey steers each weighing approximately 200 kg, were fed *ad libitum* on hay and straw of adequate zinc (26.4 ± 3.4ppm) but low selenium (0.026ppm) concentration. Sodium monensin supplements (125mg orally per day) were given to two of the steers and two left untreated and then the treatments were crossed over. Selenium or zinc absorption was assessed using the Compton whole body counter to daily monitor the amount of radioactivity retained in each animal over a period of at least two weeks after a single oral dose of Na₂⁷⁵SeO₃ or ⁶⁵ZnCl₂. Selenium absorption increased from a mean of 13.4 ± 0.8% in untreated steers to 23.2 ± 1.8% in monensin supplemented steers and from 6.3 ± 0.3% to 9.0 ± 0.3% respectively in the case of zinc absorption. In another experiment eight Friesian steers approximately 9 months old were fed *ad libitum* on hay and calf nuts adequate for both zinc and selenium and half received daily oral supplements of narasin (25ppm). Selenium absorption increased from a mean of 12.8 ± 1.3% in the untreated group to 24.0 ± 1.3% in the narasin supplemented group.

The results show that the absorption of selenium from the diet is almost doubled in steers given supplements of either monensin or narasin and that zinc absorption is significantly (P<0.001) increased in steers given monensin. We therefore suggest that, in addition to their functions as growth promotants in cattle and as coccidiostats in a number of species, ionophoric feed additives may be useful modifiers of trace element metabolism in ruminant animals.

### Example 3

Selenium metal and barium selenate were used as a source of selenium for incorporation into CRC devices.

### A. Se Metal

CRC devices were prepared with the following fomulation:

| Formulation 1 | |
|---|---|
| HGDS | 56.85 % |
| Monensin sodium | 42.37 % |
| Se metal | 0.78 % |

| Formulation 2 | |
|---|---|
| HGDS | 56.40 % |
| Monensin sodium | 42.04 % |
| Se metal | 1.56 % |

Each core weighed 75g and was 110mm long. Hence cores from Formulation 1 contained 5.32mg Se metal per mm core length, and Formulation 2 contained 10.6mg Se metal per mm core length.

*In vitro* testing of the devices showed that Formulation 1 devices payed out at 0.43mm/day and Formulation 2 at 0.48mm/day.

Hence payout from the two formulations were respectively
2.3mg of Se and 124mg of monensin sodium per day
5.1mg of Se and 138mg of monensin sodium per day.
for Formulation 1 and 2.

### B. Barium selenate

CRC devices were prepared with the following formulations:

| Formulation 3 | |
|---|---|
| HGDS | 51.46 % |
| Monensin sodium | 45.60 % |
| Barium selenate | 2.94 % |

| Formulation 4 | |
|---|---|
| HGDS | 48.52 % |
| Monensin sodium | 45.60 % |
| Barium selenate | 5.88 % |

| Formulation 5 | |
|---|---|
| HGDS | 42.64 % |
| Monensin sodium | 45.60 % |
| Barium selenate | 11.76 % |

Each core contained 75g and was 110mm long. Hence cores from:
Formulation 3 contained 5.55mg Se metal per mm core length
Formulation 4 contained 11.11mg Se metal per mm core length and
Formulation 5 contained 22.22mg Se metal per mm core length

*In vitro* payout of the devices showed that Formulation 3 delivered 0.57mm core/day and thus 3.2mg of Se and 177mg monensin sodium/day.

Formulation 4 delivered 0.52mm core/day and 5.8mg Se and 161mg monensin sodium/day, and Formulation 5 delivered 0.49mm core/day and therefore 10.9mg Se and 152mg monensin sodium/day.

Both Se, in the form of Se metal and barium selenate, and monensin sodium can be delivered through a CRC device concurrently.

### Industrial Applicability

A formulation of the invention can be readily utilised by livestock grazing on land which is deficient in selenium. The formulation can also be readily utilised by livestock to increase their absorption of selenium.

## Claims

1. A non-therapeutic method of increasing liveweight gain in ruminant livestock comprising daily administration to said livestock of an effective amount of a formulation comprising 0.5 to 60 wt% of a polyether antibiotic selected from monensin, narasin, lasalocid, and salinomycin or an alkali metal salt or ester thereof, 0.01 to 2 wt% selenium present as selenium salt, selenide, selenate, selenite, or elemental selenium, and 40 to 99.49 wt% of a veterinary acceptable carrier, diluent, excipient and/or adjuvant said weight percents based on the total weight of the formulation.

2. The method of Claim 1 wherein the formulation comprises 0.5 to 5wt% of said polyether antibiotic, 0.01 to 2% of selenium and 93 to 99.49% of said carrier, diluent, excipients and/or adjuvant.

3. The method of Claim 1 or 2 wherein 0.5 to 10 wt% of said formulation, based on total live weight of said livestock, is administered daily.

4. The method of any Claims 1 to 3 wherein said polyether antibiotic is monensin, an alkali metal salt of monensin or a monensin ester.

5. The method of any one of Claims 1 to 4 wherein said selenium is elemental selenium and/or barium selenate.

6. The method of Claim 5 wherein said selenium is barium selenate.

7. The method of any one of Claims 1 to 5 wherein the formulation administered daily to a single animal comprises 75 to 250 mg of monensin and 1 to 10 mg selenium.

8. The method of any one of Claims 1 to 7 wherein said formulation is administered directly into feed or by way of a capsule which is inserted into the rumen of said livestock.

9. The method according to Claim 8 wherein said formulation is administered by way of a controlled release capsule which is inserted into the rumen of said livestock.

10. A controlled release capsule for insertion into the rumen of livestock, which includes a formulation comprising 0.5 to 60 wt% of a polyether antibiotic selected from monensin, narasin, lasalocid and salinomycin or an alkali metal salt or ester thereof, 0.01 to 2 wt% of selenium present as selenium salt, selenide, selenate or selenite, and 40 to 99.49 wt% of a veterinary acceptable carrier, diluent, excipient and/or adjuvant, said weight percents based on the total weight of the formulation.

11. The capsule of Claim 10 wherein said formulation comprises 0.5 to 5wt% of said polyether antibiotic, 0.01 to 2% of selenium and 93 to 99.49% of said carrier, diluent, excipients and/or adjuvant.

12. The capsule of Claim 10 or 11 wherein said selenium is present as selenate.

13. The capsule of Claim 12 wherein said selenium is present as a barium selenate.

14. The capsule of any one of Claims 10 to 13 wherein said polyether antibiotic is monensin, an alkali metal salt of monensin or a monensin ester.

15. The capsule of any one of Claims 10 to 14 which is capable of administering 0.5 to 10wt% of the formulation per day based on the total live weight of said livestock.

16. The capsule of any one of Claims 10 to 15 which is capable of administering 75 to 250 mg of said polyether antibiotic and 1 to 10 mg selenium per day.

17. Use of a capsule as defined in any one of Claims 10 to 16 in a method of increasing liveweight gain in ruminant livestock.

## Patentansprüche

1. Nicht-therapeutisches Verfahren zur Erhöhung der Lebendgewichtszunahme bei wiederkäuendem Vieh, umfassend eine tägliche Verabreichung von einer wirksamen Menge an das Vieh von einer Formulierung, umfassend 0,5 bis 60 Gew.-% eines Polyetherantibiotikums, ausgewählt aus Monensin, Narasin, Lasalocid und Salinomycin oder einem Alkalimetallsalz oder Ester davon, 0,01 bis 2 Gew.-% Selen, das vorliegt als Selensalz, Selenid, Selenat, Selenit oder elementares Selen und 40 bis 99,49 Gew.-% eines tierärztlich annehmbaren Trägers, Verdünnungsmittels, Hilfsstoffs und/oder Adjuvans, wobei die Gewichtsprozente bezogen sind auf das Gesamtgewicht der Formulierung.

2. Verfahren nach Anspruch 1, wobei die Formulierung 0,5 bis 5 Gew.-% von dem Polyetherantibiotikum, 0,01 bis 2 % an Selen und 93 bis 99,49 % von dem Träger, Verdünnungsmittel, Hilfsstoffen und/oder Adjuvans umfasst.

3. Verfahren nach Anspruch 1 oder 2, wobei 0,5 bis 10 Gew.-% der Formulierung, bezogen auf das Gesamtlebendgewicht des Viehs, täglich verabreicht wird.

4. Verfahren nach einem der Ansprüche 1 bis 3, wobei das Polyetherantibiotikum für Monensin, ein Alkalimetallsalz von Monensin oder einen Monensinester steht.

5. Verfahren nach einem der Ansprüche 1 bis 4, wobei das Selen für elementares Selen und/oder Bariumselenat steht.

6. Verfahren nach Anspruch 5, wobei das Selen für Bariumselenat steht.

7. Verfahren nach einem der Ansprüche 1 bis 5, wobei die Formulierung, die täglich einem einzelnen Tier verabreicht wird, 75 bis 250 mg an Monensin und 1 bis 10 mg Selen umfasst.

8. Verfahren nach einem der Ansprüche 1 bis 7, wobei die Formulierung direkt in das Futter oder mit Hilfe einer Kapsel verabreicht wird, die in das wiedergekäute Futter des Viehs eingefügt wird.

9. Verfahren nach Anspruch 8, wobei die Formulierung verabreicht wird mit Hilfe einer Kapsel mit kontrollierter Freisetzung, die in das wiedergekäute Futter des Viehs eingefügt wird.

10. Kapsel mit kontrollierter Freisetzung zum Einfügen in das wiedergekäute Futter von Vieh, die einschließt eine Formulierung, umfassend 0,5 bis 60 Gew.-% eines Polyetherantibiotikums, ausgewählt aus Monensin, Narasin, Lasalocid und Salinomycin oder einem Alkalimetallsalz oder Ester davon, 0,01 bis 2 Gew.-% von Selen, das vorliegt als Selensalz, Selenid, Selenat oder Selenit und 40 bis 99,49 Gew.-% eines tierärztlich annehmbaren Trägers, Verdünnungsmittels, Hilfsstoffs und/oder Adjuvans, wobei die Gewichtsprozente bezogen sind auf das Gesamtgewicht der Formulierung.

11. Kapsel nach Anspruch 10, wobei die Formulierung 0,5 bis 5 Gew.-% des Polyetherantibiotikums, 0,01 bis 2 % von Selen und 93 bis 99,49 % von dem Träger, Verdünnungsmittel, Hilfsstoffen und/oder Adjuvans umfasst.

12. Kapsel nach Anspruch 10 oder 11, wobei das Selen als Selenat vorliegt.

13. Kapsel nach Anspruch 12, wobei das Selen als ein Bariumselenat vorliegt.

14. Kapsel nach einem der Ansprüche 10 bis 13, wobei das Polyetherantibiotikum für Monensin, ein Alkalimetallsalz von Monensin oder einen Monensinester steht.

15. Kapsel nach einem der Ansprüche 10 bis 14, die in der Lage ist, 0,5 bis 10 Gew.-% der Formulierung pro Tag, bezogen auf das Gesamtgewicht des Viehs, zu verabreichen.

16. Kapsel nach einem der Ansprüche 10 bis 15, die in der Lage ist, 75 bis 250 mg des Polyetherantibiotikums und 1 bis 10 mg Selen pro Tag zu verabreichen.

17. Verwendung einer Kapsel, wie sie in einem der Ansprüche 10 bis 16 definiert ist, bei einem Verfahren zur Erhöhung der Lebendgewichtszunahme bei wiederkäuendem Vieh.

## Revendications

1. Procédé non thérapeutique pour augmenter le gain de poids du bétail ruminant comprenant une administration quotidienne audit bétail d'une quantité efficace d'une formulation comprenant 0,5 à 60% en poids d'un antibiotique polyéther choisi parmi la monensine, la narasine, le lasalocide et la salinomycine ou un sel de métal alcalin ou un ester de ceux-ci, 0,01 à 2% en poids de sélénium présent sous la forme de sel de sélénium, séléniure, séléniate, sélénite ou sélénium élémentaire, et 40 à 99,49% en poids d'un support, d'un diluant, d'un excipient et/ou d'un adjuvant vétérinairement acceptables, lesdits pourcentages en poids étant exprimés sur la base du poids total de la formulation.

2. Procédé selon la revendication 1, dans lequel la formulation comprend 0,5 à 5% en poids dudit antibiotique polyéther, 0,01 à 2% de sélénium et 93 à 99,49% desdits support, diluant, excipient et/ou adjuvant.

3. Procédé selon la revendication 1 ou 2, dans lequel 0,5 à 10% en poids de ladite formulation, sur la base du poids total dudit bétail, est (sont) administré(s) quotidiennement.

4. Procédé selon l'une quelconque des revendications 1 à 3, dans lequel ledit antibiotique polyéther est la monensine, un sel de métal alcalin de monensine ou un ester de monensine.

5. Procédé selon l'une quelconque des revendications 1 à 4, dans lequel ledit sélénium est du sélénium élémentaire et/ou du séléniate de baryum.

6. Procédé selon la revendication 5, dans lequel ledit sélénium est le séléniate de baryum.

7. Procédé selon l'une quelconque des revendications 1 à 5, dans lequel la formulation administrée quotidiennement à un seul animal comprend 75 à 250 mg de monensine et 1 à 10 mg de sélénium.

8. Procédé selon l'une quelconque des revendications 1 à 7, dans lequel ladite formulation est administrée directement dans la nourriture ou au moyen d'une gélule qui est insérée dans le rumen dudit bétail.

9. Procédé selon la revendication 8, dans lequel ladite formulation est administrée au moyen d'une gélule à libération contrôlée qui est insérée dans le rumen dudit bétail.

10. Gélule à libération contrôlée pour insertion dans le rumen du bétail, qui comprend une formulation comprenant 0,5 à 60% en poids d'un antibiotique polyéther choisi parmi la monensine, la narasine, le lasalocide et la salinomycine ou un sel de métal alcalin ou un ester de ceux-ci, 0,01 à 2% en poids de sélénium présent sous la forme de sel de sélénium, séléniure, séléniate ou sélénite, et 40 à 99,49% en poids d'un support, d'un diluant, d'un excipient et/ou d'un adjuvant vétérinairement acceptables, lesdits pourcentages en poids étant exprimés sur la base du poids total de la formulation.

11. Gélule selon la revendication 10, dans laquelle ladite formulation comprend 0,5 à 5% en poids dudit antibiotique polyéther, 0,01 à 2% de sélénium et 93 à 99,49% desdits support, diluant, excipient et/ou adjuvant.

12. Gélule selon la revendication 10 ou 11, dans laquelle ledit sélénium est présent sous la forme de séléniate.

13. Gélule selon la revendication 12, dans laquelle ledit sélénium est présent sous la forme de séléniate de baryum.

14. Gélule selon l'une quelconque des revendications 10 à 13, dans laquelle ledit antibiotique polyéther est la monensine, un sel de métal alcalin de monensine ou un ester de monensine.

15. Gélule selon l'une quelconque des revendications 10 à 14, qui est capable d'administrer 0,5 à 10% en poids de la formulation par jour, sur la base du poids total dudit bétail.

16. Gélule selon l'une quelconque des revendications 10 à 15, qui est capable d'administrer 75 à 250 mg dudit antibiotique polyéther et 1 à 10 mg de sélénium par jour.

17. Utilisation d'une gélule telle que définie dans l'une quelconque des revendications 10 à 16 dans un procédé destiné à augmenter le gain de poids du bétail ruminant.
